(19) Europäisches Patentamt
European Patent Office
Office européen des brevets

(11) Veröffentlichungsnummer: **0 138 177**
**B1**

(12) # EUROPÄISCHE PATENTSCHRIFT

(45) Veröffentlichungstag der Patentschrift: **07.01.88**

(51) Int. Cl.[4]: **C 07 D 493/10**, B 41 M 5/12, B 41 M 5/26 // (C07D493/10, 311:00, 307:00)

(21) Anmeldenummer: **84112011.6**

(22) Anmeldetag: **06.10.84**

(54) **Fluorane und deren Verwendung.**

(30) Priorität: **14.10.83 DE 3337387**

(43) Veröffentlichungstag der Anmeldung: **24.04.85 Patentblatt 85/17**

(45) Bekanntmachung des Hinweises auf die Patenterteilung: **07.01.88 Patentblatt 88/1**

(84) Benannte Vertragsstaaten: **CH DE FR GB IT LI**

(56) Entgegenhaltungen: **GB - A - 2 014 629**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Mayer, Udo, Dr., Max-Slevogt-Strasse 27, D-6710 Frankenthal (DE)**
Erfinder: **Oberlinner, Andreas, Dr., Bruesseler Ring 53, D-6700 Ludwigshafen (DE)**

EP 0 138 177 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Fluorane und deren Verwendung in Aufzeichnungssystemen.

Fluorane und deren Verwendung in Aufzeichnungssystemen sind bekannt (z.B. DE-OS 2 039 848, 2 422 899 und 2 024 859, DE-AS 1 671 545 und 1 543 803, GB-PS 1 211 393 und 2 014 629 und US-PS 3 506 471 und 3 959 571).

Aufgabe der Erfindung war es, besser lösliche und in der Migration verbesserte Fluorane aufzufinden.

Diese Aufgabe wird durch die Fluorane der Erfindung gelöst.

Dementsprechend betrifft die Erfindung Fluorane der allgemeinen Formel I

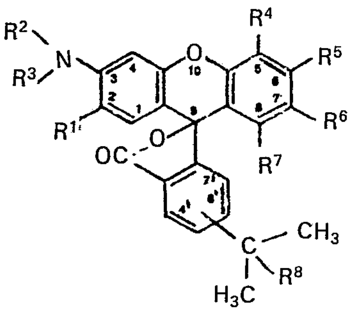

(I),

in der

$R^1$ für Wasserstoff oder Methyl,

$R^2$ für Wasserstoff, $C_1$- bis $C_4$-Alkyl, $C_7$- oder $C_8$-Phenalkyl, Cyanethyl, Chlorethyl oder Alkoxyalkyl mit insgesamt 3 bis 5 C-Atomen,

$R^3$ für Wasserstoff, $C_1$- bis $C_4$-Alkyl, $C_7$- bis $C_{10}$-Phenalkyl, $C_5$- oder $C_6$-Cycloalkyl, Cyanethyl, Chlorethyl, Alkoxyalkyl mit insgesamt 3 bis 5 C-Atomen oder für gegebenenfalls durch $C_1$- bis $C_4$-Alkyl, Methoxy, Chlor oder Brom substituiertes Phenyl, oder $-N\begin{matrix}R^2\\R^3\end{matrix}$ für N-Pyrrolidinyl, N-Piperidinyl, N-Morpholinyl oder N-Isoindolinyl,

$R^4$ und $R^5$ unabhängig voneinander für Wasserstoff, $C_1$- bis $C_4$-Alkyl, $C_1$- bis $C_4$-Alkoxy oder Halogen,

$R^6$ für Wasserstoff, Halogen, $C_1$- bis $C_{12}$-Alkyl, $C_1$- bis $C_4$-Alkoxy, $C_7$- bis $C_{10}$-Phenalkoxy, Phenoxy, Phenyl, oder für eine Gruppe der Formel

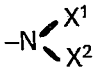

worin $X^1$ Wasserstoff, $C_1$- bis $C_4$-Alkyl, Chlorethyl, Alkoxyalkyl mit insgesamt 3 bis 5 C-Atomen, $C_7$- oder $C_8$-Phenalkyl und $X^2$ Wasserstoff, $C_1$- bis $C_{12}$-Alkyl, Chlorethyl, $C_5$- oder $C_6$-Cycloalkyl, Alkoxyalkyl mit insgesamt 3 bis 5 C-Atomen, $C_7$- bis $C_{10}$-Phenalkyl oder gegebenenfalls durch Halogen, $C_1$- bis $C_4$-Alkyl, $C_1$- bis $C_4$-Alkoxy, $C_1$- bis $C_4$-Alkylcarbonyl, Benzoyl oder Phenoxy substituiertes Phenyl, $C_1$- bis $C_4$-Alkoxycarbonyl-$C_1$- bis $C_3$-alkyl oder Carbamoyl-$C_1$- bis $C_3$-alkyl bedeuten, oder 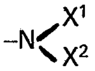 N-Pyrrolidinyl, N-Piperidinyl oder N-Morpholinyl bedeuten,

$R^7$ für Wasserstoff, $C_1$- bis $C_4$-Alkyl oder Halogen und

$R^8$ für $C_1$- bis $C_5$-Alkyl stehen und wobei die Reste $R^4$ und $R^5$ und/oder $R^6$ und $R^7$ gemeinsam je eine $-CH=CH-CH=CH$-Brücke sein können und der ankondensierte Ring gegebenenfalls ein Halogen oder eine gegebenenfalls durch $C_1$- bis $C_4$-Alkyl, $C_7$- oder $C_8$-Phenalkyl mono- oder disubstituierte Aminogruppe tragen kann.

Die erfindungsgemässen Fluorane zeichnen sich durch eine verbesserte Löslichkeit in den üblicherweise bei der Herstellung von Mikrokapseln verwendeten (Kern) Lösungsmitteln aus. Sie erlauben daher eine grössere Flexibilität bei der Wahl der Lösungsmittel. Da die Verwendung Halogen enthaltender Lösungsmittel, welche die Fluorane des Standes der Technik gut lösen, für Mikrokapseln jedoch aus Gründen der Toxizität und der Ökologie auf Bedenken stösst, ist die universelle bessere Löslichkeit der erfindungsgemässen Fluorane in anderen aus physiologischer Sicht unbedenklicheren Lösungsmitteln von grosser Bedeutung. Ausserdem weisen eine Reihe der neuen Fluorane eine geringere Migration im Trägermaterial auf als die Fluorane des Standes der Technik.

Die Fluorane der allgemeinen Formel I sind schwach farbige bis farblose Verbindungen, deren Lösungen in inerten organischen Lösungsmitteln im Kontakt mit Elektronenakzeptoren Färbungen in gelben, orangenen, roten, blauen, oliven oder schwarzen Farbtönen geben, je nach der Substitution des Fluorans. Beispiele für Elektronenakzeptorsubstanzen sind Carbon- oder Mineralsäuren, Kaolin, Bentonit, aktivierter Ton, Aluminiumsilikat, Attapulgit oder jeder beliebige Ton, sauer reagierende polymere Materialien wie Kondensationsprodukte auf der Basis Phenolen und/oder Phenolsulfonsäuren, ferner Metalloxide oder -salze wie Zinkoxid, Aluminiumoxid, Zinkchlorid, Eisenstearat oder Cobaltnaphthenat.

Aufgrund dieser Eigenschaften sind die neuen Verbindungen (I) als Farbbildner zur Verwendung in druck- und wärmeempfindlichen Aufzeichnungsmaterialien geeignet.

Für die Anwendung in druckempfindlichen Systemen werden die Fluorane (I) vorteilhafterweise in Form von Lösungen in organischen Lösungsmitteln wie Chlorparaffinen, partiell hydriertem Di- oder Terphenyl, Alkylbenzolen, Alkylnaphthalinen, alkyliertem Dibenzylbenzol, Paraffinöl, Mineralöl oder auch in üblichen tiefer siedenden Lösungsmitteln wie Xylol oder Toluol in Mikrokapseln eingeschlossen und mit diesen der Träger, z.B. Papier beschichtet. Bei Druck tritt dann im Kontakt mit Elektronenakzeptoren an der Druckstelle Farbbildung ein.

Geeignete Verfahren zur Herstellung von Mikrokapseln sind z.B. aus den US-PS 2 800 457 und 2 800 458, der DE-PS 2 119 933 und der EP-PS 26 914 bekannt. Man kann die erfindungsgemässen Verbindungen der allgemeinen Formel I auch nach dem in der US-PS 3 103 404 beschriebenen Verfahren in Wachs oder Öl-Wachsmischungen fein verteilen und mit diesen Mischungen Träger, wie Folien oder Papier beschichten. Man erhält so druckempfindliche Materialien, die zum Durchschreiben auf mit Elektronenakzeptorsubstanzen beschichteten Papieren geeignet sind und die nach Gebrauch wie Kohlepapier entfernt werden.

Die erfindungsgemässen Fluorane können auch als Farbbildner in wärmeempfindlichen Aufzeichnungsmaterialien verwendet werden, die auf einem Träger ein Bindemittel, einen Farbbildner und eine Elektronenakzeptorsubstanz enthalten. Der Aufbau solcher wärmeempfindlicher Aufzeichnungsmaterialien und die Zusammensetzung der durch Wärmeeinfluss die Farbe erzeugenden Schichten sind bekannt (z.B. DE-OS 2 228 581, DE-OS 2 110 854) ebenso die Verfahren und Vorrichtungen mit denen die Farbbildung erreicht wird.

Als Substituenten $R^1$ bis $R^8$ kommen für die Fluorane der allgemeinen Formel I im einzelnen z.B. in Betracht:

$R^1$: Methyl, vorzugsweise Wasserstoff.

Als $C_1$- bis $C_4$-Alkyl kommen für $R^2$, $R^3$, $R^4$, $R^5$, $R^7$ und $X^1$ unabhängig voneinander z.B. im einzelnen in Betracht: Methyl, Ethyl, n- und i-Propyl, n- und i-Butyl.

Für $R^6$ und $X^2$ sind als $C_1$- bis $C_{12}$-Alkyl z.B. neben den für $C_1$- bis $C_4$-Alkyl bereits im einzelnen genannten Resten jeweils n-Pentyl, n-Hexyl, n-Octyl, 2-Ethylhexyl, Nonyl, Decyl und Dodecyl zu nennen.

$C_1$- bis $C_5$-Alkylreste $R^8$ sind z.B. Methyl, Ethyl, Propyl, n-Butyl, sec.-Butyl, Pentyl, 2,2-Dimethylpropyl-1, von denen Ethyl und 2,2-Dimethylpropyl-1 und vor allem Methyl bevorzugt sind.

Als $C_1$- bis $C_4$-Alkoxy kommen für $R^4$ und $R^5$ z.B. Methoxy, Ethoxy, n- und i-Propoxy, n- und i-Butoxy in Betracht.

Für $R^2$, $R^3$, $X^1$ und $X^2$ kommen als Alkoxyalkyl mit 3 bis 5 C-Atomen z.B. in Betracht: 2-Methoxyethyl, 2-Ethoxyethyl, 2-Propoxyethyl, 3-Methoxypropyl und 3-Ethoxypropyl.

Für $R^2$ und $X^1$ sind als Phenalkyl Benzyl, 1- und 2-Phenylethyl im einzelnen zu nennen.

Als $C_7$- bis $C_{10}$-Phenalkyl kommen für $R^3$, $R^6$ und $X^2$ neben den für $R^2$ genannten Phenalkyl z.B. in Betracht: 2- und 3-Phenylpropyl, 2-, 3- und 4-Phenylbutyl.

Als $C_5$- und $C_6$-Cycloalkyl kommen für $R^3$ und $X^2$ jeweils z.B. Cyclopentyl, Methylcyclopentyl und vorzugsweise Cyclohexyl in Betracht.

Halogen steht für Chlor, Brom oder Fluor, vorzugsweise für Brom und insbesondere für Chlor.

$R^3$ kann ausserdem ein gegebenenfalls durch $C_1$- bis $C_4$-Alkyl, Methoxy, Chlor oder Brom substituiertes Phenyl sein, wobei als $C_1$- bis $C_4$-Alkyl die für $R^2$ genannten Reste in Betracht kommen.

$X^2$ kann auch für ein gegebenenfalls durch Halogen, $C_1$- bis $C_4$-Alkyl, $C_1$- bis $C_4$-Alkoxy, $C_1$- bis $C_4$-Alkylcarbonyl, Benzoyl oder Phenoxy substituiertes Phenyl stehen. Als $C_1$- bis $C_4$-Alkyl kommen die für $R^2$ und als $C_1$- bis $C_4$-Alkoxy die für $R^4$ genannten Reste z.B. in Betracht. Als $C_1$- bis $C_4$-Alkylcarbonyl sind z.B. Acetyl, Propionyl, Butyroyl und Valeroyl (Pentanoyl) zu nennen.

Daneben kann $X^2$ auch für $C_1$- bis $C_4$-Alkoxycarbonyl-$C_1$- bis $C_3$-alkyl oder für Carbamoyl-$C_1$- bis $C_3$-alkyl stehen, wofür im einzelnen z.B. Methoxycarbonylmethyl, Ethoxycarbonylmethyl, Ethoxycarbonylethyl, Carbamoylmethyl oder Carbamoylethyl zu nennen sind.

Aus anwendungstechnischen Gründen sind Fluorane der allgemeinen Formel I bevorzugt, in denen $R^8$ für Methyl, Ethyl oder 2,2-Dimethylpropyl-1 steht. Von diesen sind die Fluorane mit $R^8 = CH_3$ ganz besonders bevorzugt.

Aus anwendungstechnischer Sicht sind Fluorane der allgemeinen Formeln (II), (III), (IV) und (V) bevorzugt:

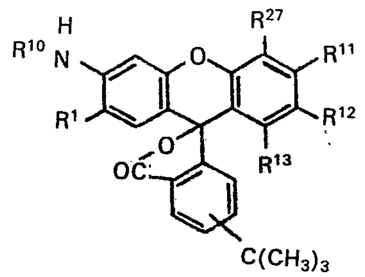

(II),

in der

$R^1$ für Wasserstoff oder Methyl,

$R^{10}$ für Wasserstoff, $C_1$- bis $C_4$-Alkyl, Cyclohexyl, Benzyl oder für gegebenenfalls durch ein oder zwei Methyl und/oder Ethyl und/oder ein Chlor, Brom oder Methoxy substituiertes Phenyl,

$R^{11}$ für Wasserstoff, Chlor oder $C_1$- bis $C_4$-Alkyl,

$R^{12}$ für $C_1$- bis $C_{12}$-Alkyl oder Halogen,

$R^{13}$ für Wasserstoff oder Methyl und

$R^{27}$ für Wasserstoff oder Methyl stehen.

Vorzugsweise steht $R^1$ für Wasserstoff wenn $R^{10}$ für gegebenenfalls substituiertes Phenyl steht.

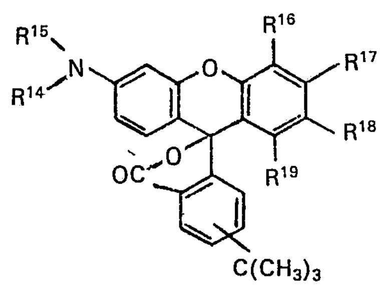

(III),

in der

$R^{14}$ für $C_1$- bis $C_4$-Alkyl, $C_7$- oder $C_8$-Phenalkyl oder Alkoxyalkyl mit insgesamt 3 bis 5 C-Atomen,

$R^{15}$ für $C_1$- bis $C_4$-Alkyl, $C_7$- bis $C_{10}$-Phenalkyl, $C_5$- oder $C_6$-Cycloalkyl, Alkoxyalkyl mit insgesamt 3 bis 5 C-Atomen oder für gegebenenfalls durch $C_1$- bis $C_4$-Alkyl, Chlor oder Brom einfach substituiertes Phenyl oder $-N{<}^{R^{14}}_{R^{15}}$ für N-Pyrrolidinyl, N-Piperidinyl oder N-Morpholinyl,

$R^{16}$ und $R^{17}$ unabhängig voneinander für Wasserstoff, Chlor oder $C_1$- bis $C_4$-Alkyl,

$R^{18}$ für Wasserstoff, $C_1$- bis $C_{12}$-Alkyl, $C_1$- bis $C_4$-Alkoxy, $C_7$- bis $C_{10}$-Phenalkoxy, Phenoxy oder Halogen und

$R^{19}$ für Wasserstoff oder $C_1$- bis $C_4$-Alkyl stehen.

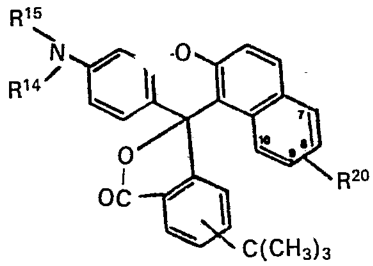

(IV),

in der $R^{14}$ und $R^{15}$ die vorstehend angegebenen Bedeutungen haben und $R^{20}$ für Wasserstoff oder für eine gegebenenfalls durch Methyl, Ethyl oder Benzyl mono- oder disubstituierte Aminogruppe in der 7-, 9- oder 10-Stellung steht.

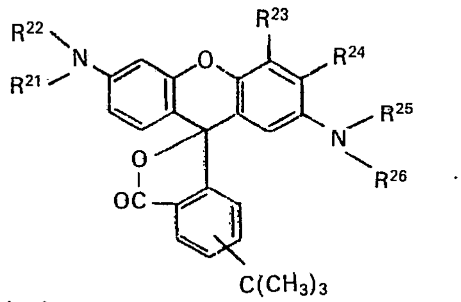

(V),

in der

$R^{21}$ für $C_1$- bis $C_4$-Alkyl, $C_7$- oder $C_8$-Phenalkyl,

$R^{22}$ für $C_1$- bis $C_4$-Alkyl, $C_7$- bis $C_{10}$-Phenalkyl, $C_5$- oder $C_6$-Cycloalkyl oder für durch $C_1$- bis $C_4$-Alkyl oder Chlor substituiertes Phenyl oder $-N{<}^{R^{21}}_{R^{22}}$ für N-Pyrrolidinyl, N-Piperidinyl oder N-Morpholinyl,

$R^{23}$ für Wasserstoff oder Chlor,

$R^{24}$ für Wasserstoff oder Methyl, wobei wenn $R^{23}$ Wasserstoff ist, $R^{24}$ auch Chlor sein kann,

$R^{25}$ für Wasserstoff, $C_1$- bis $C_4$-Alkyl, $C_7$- oder $C_8$-Phenalkyl und $R^{26}$ für Wasserstoff, $C_1$- bis $C_{12}$-Alkyl, $C_7$- bis $C_{10}$-Phenalkyl, $C_5$- oder $C_6$-Cycloalkyl oder für ein gegebenenfalls durch Chlor, $C_1$- bis $C_4$-Alkyl, Methoxy, Ethoxy, $C_1$- bis $C_2$-Alkylcarbonyl, Benzoyl oder Phenoxy substituiertes Phenyl oder $-N{<}^{R^{25}}_{R^{26}}$ für N-Pyrrolidinyl, N-Piperidinyl oder N-Morpholinyl stehen.

Aus anwendungstechnischen Gründen sind besonders hervorzuheben:

a) Fluorane der allgemeinen Formel II mit

| | $R^1$ | $R^{10}$ | $R^{11}$ | $R^{12}$ | $R^{13}$ | $R^{27}$ |
|---|---|---|---|---|---|---|
| a1) | $CH_3$ | $C_2H_5$ | H | $CH_3$ | H | H |
| a2) | $CH_3$ | $C_2H_5$ | $CH_3$ | $CH_3$ | H | H |
| a3) | $CH_3$ | $C_2H_5$ | H | $CH_3$ | $CH_3$ | H |
| a4) | H | 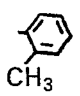 | $CH_3$ | $CH_3$ | H | H |
| a5) | H | 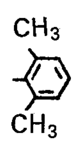 | $CH_3$ | $CH_3$ | H | H |
| a6) | H | 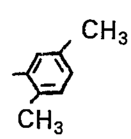 | $CH_3$ | $CH_3$ | H | H |
| a7) | H | 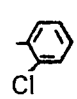 | $CH_3$ | $CH_3$ | H | H |
| a8) | H | 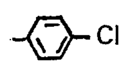 | $CH_3$ | $CH_3$ | H | H |

a) Fluorane der allgemeinen Formel II mit

| | $R^1$ | $R^{10}$ | $R^{11}$ | $R^{12}$ | $R^{13}$ | $R^{27}$ |
|---|---|---|---|---|---|---|
| a9) | H | 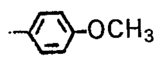 | $CH_3$ | $CH_3$ | H | H |
| a10) | $CH_3$ | $C_2H_5$ | H | $CH_3$ | H | $CH_3$ |
| a11) | $CH_3$ | $C_2H_5$ | $CH_3$ | Cl | H | H |
| a12) | $CH_3$ | $C_2H_5$ | H | Cl | H | H |
| a13) | H | 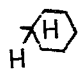 | Cl | H | H | H |

b) Fluorane der allgemeinen Formel III mit

| | 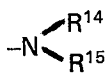 | $R^{16}$ | $R^{17}$ | $R^{18}$ | $R^{19}$ |
|---|---|---|---|---|---|
| b1) | $N(CH_3)_2$ | H | H | $C(CH_3)_3$ | H |
| b2) | $N(C_2H_5)_2$ | H | H | $C(CH_3)_3$ | H |
| b3) | 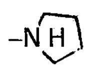 | H | H | $C(CH_3)_3$ | H |
| b4) | 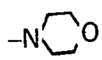 | H | H | $C(CH_3)_3$ | H |
| b5) | $N(CH_3)_2$ | H | H | Cl | H |
| b6) | $N(C_2H_5)_2$ | H | H | Cl | H |
| b7) | $N(C_2H_5)_2$ | H | H | $OCH_3$ | H |
| b8) | $N(C_2H_5)_2$ | H | H | 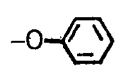 | H |

c) Fluorane der allgemeinen Formel IV mit

| | 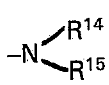 | $R^{20}$ |
|---|---|---|
| c1) | $N(CH_3)_2$ | H |
| c2) | $N(C_2H_5)_2$ | H |
| c3) | 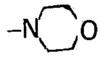 | H |

d) Fluorane der allgemeinen Formel V mit

| | $R^{22}R^{21}>N-$ | $R^{23}$ | $R^{24}$ | $R^{26}$ | $R^{25}$ |
|---|---|---|---|---|---|
| d1) | $(CH_3)_2N$ | H | $CH_3$ | 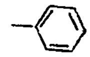 | H |
| d2) | $(C_2H_5)_2N$ | H | $CH_3$ | 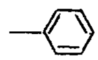 | H |
| d3) | | H | $CH_3$ | 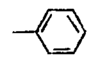 | H |
| d4) | $(C_2H_5)_2N$ | H | $CH_3$ | 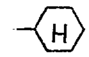 | H |
| d5) | $(C_2H_5)_2N$ | H | H | 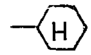 | H |

Die Verbindungen der allgemeinen Formel I können nach an sich bekannten Verfahren hergestellt werden.

Die Synthese kann z.B. durch Umsetzen von o-Hydroxybenzophenonderivaten der allgemeinen Formel VI

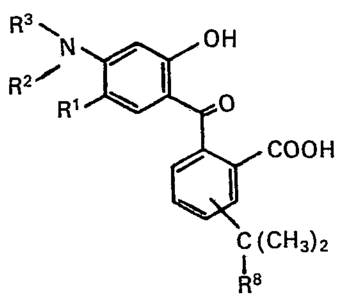

(VI)

mit Phenolderivaten der allgemeinen Formel VII

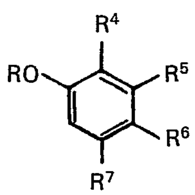

(VII)

oder von o-Hydroxybenzophenonen der allgemeinen Formel VIII

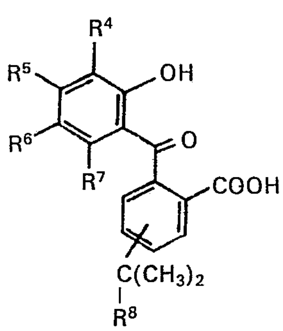

(VIII)

mit Anilinverbindungen der allgemeinen Formel IX

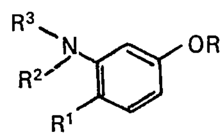

(IX)

erfolgen, wobei in den allgemeinen Formeln VII und IX R für Wasserstoff oder $C_1$- bis $C_4$-Alkyl, vorzugsweise für Wasserstoff, Methyl oder Ethyl steht und in den allgemeinen Formeln VI bis IX $R^1$ bis $R^8$ die oben angegebene Bedeutung haben.

Die Umsetzung erfolgt zweckmässigerweise bei 10 bis 130 °C, wobei die Komponenten in Gegenwart eines sauren Kondensationsmittels wie Essigsäure, Acetanhydrid, Schwefelsäure, Zinkchlorid oder Phosphoroxichlorid zur Reaktion gebracht werden.

Die Benzophenonderivate (VI) und (VIII) werden nach an sich bekannten Verfahren durch Umsetzen von Phthalsäureanhydriden der allgemeinen Formel X

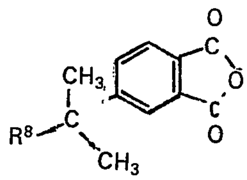

(X)

mit einer Anilinverbindung der allgemeinen Formel IX bzw. mit einem Phenol der allgemeinen Formel VII erhalten. Die Umsetzung erfolgt vorteilhafterweise in inerten organischen Lösungsmitteln wie Toluol, Xylol, Chlorbenzol oder Dichlorbenzol gegebenenfalls in Gegenwart von Kondensationsmitteln wie Zinkchlorid oder Aluminiumchlorid. Bei der Umsetzung werden Gemische aus den 4- und 5-tert. Butylbenzophenonverbindungen erhalten.

Nähere Angaben zur Herstellung der Fluorane sind den folgenden Beispielen zu entnehmen.

Die in den Beispielen genannten Teile und Prozentangaben beziehen sich auf das Gewicht.

Beispiel 1

Zu der Lösung von 46,5 Teilen eines Gemisches aus 2-(2'-Hydroxy-5'-methylbenzoyl)-4-tert.-butylbenzoesäure (erhalten durch Umsetzen von 4-tert.-Butylphthalsäureanhydrid mit 4-Methylphenol) in 160 Teilen Chloroform werden 16 Teile Phosphoroxichlorid getropft. Das Gemisch wird 4 h bei Raumtemperatur gerührt, dann werden 22,5 Teile 3-Ethylamino-4-methylphenol zugegeben und die Mischung 3 h auf Rückflusstemperatur erwärmt. Nach dem Zugeben von 150 Teilen Wasser wird das Chloroform abdestilliert und der ölige Rückstand in 100 Teilen 25%igem Ammoniakwasser und 100 Teilen Toluol gerührt. Die Toluolphase wird abgetrennt und auf $^1/_5$ des Volumens eingeengt.

Durch Zugeben von 26 Teilen Methanol werden 26 Teile 2,7-Dimethyl-3-ethylamino-5'(6')-tert.-butylfluoran gefällt:

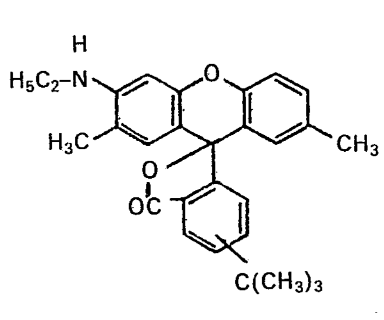

Schmp. 231 – 233 °C

Wird eine Lösung des Fluorans in partiell hydriertem Terphenyl in Mikrokapseln eingeschlossen, z.B. nach dem Verfahren der EP-B 26 914, Beispiel 1, und die Mikrokapseln auf Papier aufgebracht, dann erhält man beim Auflegen auf einer Nehmerseite beim Beschriften eine intensive orangefarbene Durchschrift.

Beispiel 2

99 Teile 2-(2'-Hydroxy-4', 5'-dimethyl-benzoyl)-4(5)-tert.-butylbenzoesäure und 45 Teile 3-Ethylamino-4-methyl-phenol werden analog den Angaben in Beispiel 1 zum Farbbildner der Formel

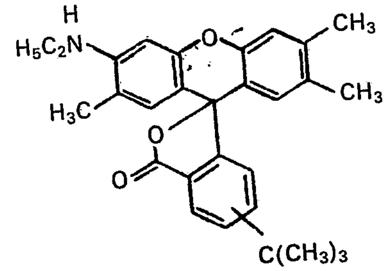

umgesetzt.

Ausbeute: 20 Teile 2,6,7-Trimethyl-3-ethylamino-5'(6')-tert.-butylfluoran. Schmp. 247–249 °C. Die Substanz entwickelt mit Elektronenakzeptorsubstanzen eine Orangefärbung.

Beispiele 3 bis 16

Analog den Angaben in Beispiel 1 werden Farbbildner der allgemeinen Formel Ia

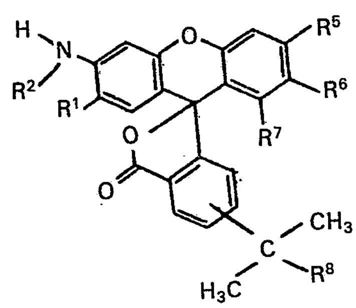

(Ia)

erhalten. Die Bedeutung der Substituenten ist in der folgenden Tabelle angegeben. Der mit Elektronenakzeptorsubstanzen gebildete Farbton ist in der rechten Spalte angegeben.

| Bsp. Nr. | $R^1$ | $R^2$ | $R^5$ | $R^6$ | $R^7$ | $R^8$ | Farbton | Schmp. [°C] |
|---|---|---|---|---|---|---|---|---|
| 3 | $CH_3$ | $C_2H_5$ | H | $CH_3$ | H | $C_2H_5$ | orange | |
| 4 | $CH_3$ | $C_2H_5$ | H | $CH_3$ | H | $CH_2C(CH_3)_3$ | orange | |
| 5 | $CH_3$ | $C_2H_5$ | $C_2H_5$ | H | $C_2H_5$ | $CH_3$ | orange | |
| 6 | $CH_3$ | $C_2H_5$ | H | $C(CH_3)_3$ | H | $CH_3$ | orange | 220–222 |
| 7 | $CH_3$ | H | H | $CH_3$ | H | $CH_3$ | gelborange | |
| 8 | $CH_3$ | $C_2H_5$ | Cl | H | H | $CH_3$ | rotorange | |
| 9 | H | $i{-}C_3H_7$ | H | $CH_3$ | H | $CH_3$ | orange | |
| 10 | H | $i{-}C_6H_{11}$ | H | $CH_3$ | H | $CH_3$ | orange | |
| 11 | H | 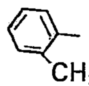 | H | $CH_3$ | H | $CH_3$ | rotbraun | |
| 12 | H | 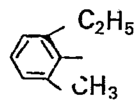 | $CH_3$ | $CH_3$ | H | $CH_3$ | rotorange | |
| 13 | H | 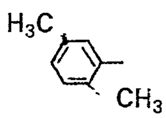 | $CH_3$ | $CH_3$ | H | $CH_3$ | rotorange | |
| 14 | H | 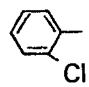 | $CH_3$ | $CH_3$ | H | $CH_3$ | rotorange | |
| 15 | H | 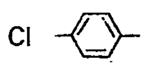 | $CH_3$ | $CH_3$ | H | $CH_3$ | rot | |
| 16 | H | 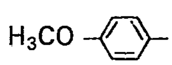 | $CH_3$ | $CH_3$ | H | $CH_3$ | rot | |
| 17 | $CH_3$ | $C_2H_5$ | H | Cl | H | $CH_3$ | orange | 226–228 |
| 18 | H | Cyclohexyl | Cl | H | H | $CH_3$ | orange | 296–298 |
| 19 | H | 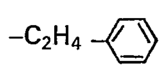 | $CH_3$ | Cl | H | $CH_3$ | rotorange | 240–242 |
| 20 | $CH_3$ | $C_2H_5$ | $CH_3$ | H | H | $CH_3$ | orange | |

Beispiel 21

34 Teile 2-(2'-Hydroxy-4'-dimethylaminobenzoyl)-4(5)-tert.-butyl-benzoesäure und 15 Teile 4-tert.-Butylphenol werden in 100 Teilen 80%iger Schwefelsäure 6 h auf 100 °C erhitzt. Das Reaktionsgemisch wird auf 200 Teile Eis und 200 Teile Methanol ausgetragen und mit 25%igem Ammoniakwasser alkalisch gestellt. Das kristalline Produkt wird isoliert und zur weiteren Reinigung in 250 Teilen Toluol, 200 Teilen Wasser und 50 Teilen 25%igem Ammoniakwasser gerührt. Die Toluolphase wird abgetrennt und auf ein Fünftel ihres ursprünglichen Volumens eingeengt. Durch Zugabe von 50 Teilen Ethanol fällt man aus dieser Lösung 13 Teile 3-Dimethylamino-7,5'(6')-bis-tert.-butylfluoran der Formel

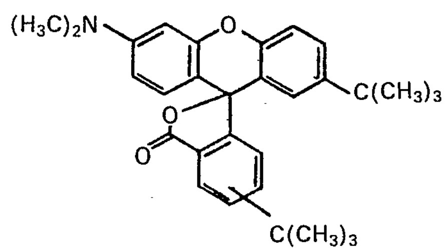

Schmp. 185 bis 187 °C. Auf Elektronenakzeptorsubstanzen erhält man eine Rotorangefärbung.

Beispiel 22

34 Teile 2-(2'-Hydroxy-4'-dimethylaminobenzoyl)-4(5)-tert.-butyl-benzoesäure und 11 Teile 4-Methyl-phenol werden analog den Angaben in Beispiel 21 umgesetzt und isoliert. Ausbeute: 21

Teile 3-Dimethylamino-7-methyl-5'(6')-tert.-butyl-fluoran der Formel

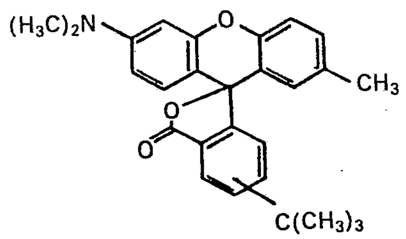

Schmp. 135–140 °C. Auf Elektronenakzeptorsubstanzen erhält man eine Rotorangefärbung.

Beispiel 23

17 Teile 2-(2'-Hydroxy-4'-dimethylaminobenzoyl)-4(5)-tert.-butyl-benzoesäure und 7,5 Teile 2-tert.-Butyl-phenol werden analog den Angaben in Beispiel 21 umgesetzt und isoliert. Ausbeute: 4 Teile 3-Dimethylamino-5,5'(6')-bis-tert.-butyl-fluoran der Formel

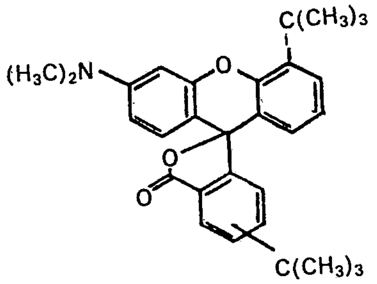

Schmp. 197–199 °C. Im Kontakt mit sauer reagierenden Substanzen erhält man eine Rotorangefärbung.

Beispiel 24

37 Teile 2-(2'-Hydroxy-4'-diethylaminobenzoyl)-4(5)-tert.-butyl-benzoesäure und 12 Teile 3,4-Dimethylphenol werden analog den Angaben in Beispiel 21 umgesetzt. Ausbeute: 14 Teile 3-Diethylamino-6(8),7-dimethyl-5'(6')-tert.-butyl-fluoran der Formel

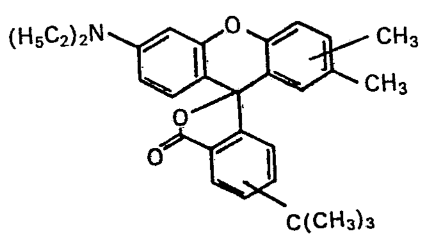

Schmp. 171–173 °C. Im Kontakt mit sauer reagierenden Substanzen erhält man eine Rotorangefärbung.

Beispiel 25

18,5 Teile 2-(2'-Hydroxy-4'-diethylaminobenzoyl)-4(5)-tert.-butyl-benzoesäure und 7,5 Teile 3,5-Diethylphenol werden analog den Angaben in Beispiel 21 zu 2,5 Teilen 3-Diethylamino-6,8-diethyl-5'(6')-tert.-butyl-fluoran der Formel

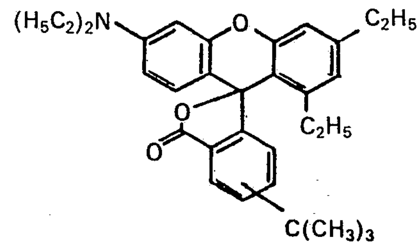

umgesetzt. Schmp. 185–186 °C. Das Fluoran entwickelt mit Elektronenakzeptorsubstanzen eine Rotorangefärbung und zeigt im Vergleich zu den nicht durch eine tert.-Butylgruppe substituierten Verbindung eine deutlich geringere Migrationsneigung.

Beispiel 26

18 Teile 2-(2'-Hydroxy-4'-diethylaminobenzoyl)-4(5)-tert.-butyl-benzoesäure und 6 Teile p-Chlorphenol werden entsprechend den Angaben in Beispiel 21 umgesetzt. Ausbeute: 7 Teile 3-Diethylamino-7-chlor-5'(6')-tert.-butyl-fluoran der Formel

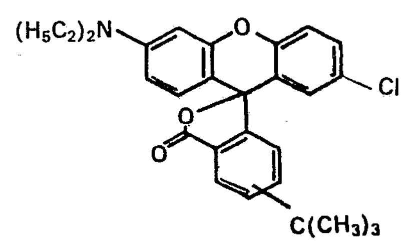

Schmp. 180–182 °C. Im Kontakt mit Elektronenakzeptorsubstanzen erhält man eine Rotfärbung.

Beispiel 27

37 Teile 2-(2'-Hydroxy-4'-diethylaminobenzoyl)-4(5)-tert.-butyl-benzoesäure und 18 Teile p-Phenoxy-phenol werden analog den Angaben in Beispiel 21 umgesetzt. Ausbeute: 3 Teile 3-Di-

ethylamino-7-phenoxy-5'(6')-tert.-butyl-fluoran der Formel

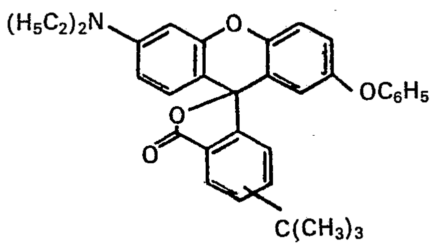

Schmp. 195–198 °C. Im Kontakt mit Elektronenakzeptorsubstanzen erhält man eine Rotfärbung.

Beispiel 28

37 Teile 2-(2'-Hydroxy-4'-diethylaminobenzoyl)-4(5)-tert.-butyl-benzoesäure und 14 Teile 3-Methyl-4-chlor-phenol werden analog den Angaben in Beispiel 21 umgesetzt. Ausbeute: 5 Teile 3-Diethylamino-6(8)-methyl-7-chlor-5'(6')-tert.-butyl-fluoran der Formel

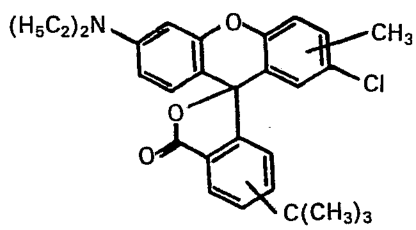

Schmp. 206–211 °C. Im Kontakt mit Elektronenakzeptorsubstanz erhält man eine Rotorangefärbung.

Beispiel 29

37 Teile 2-(2'-Hydroxy-4'-pyrrolidinobenzoyl)-4(5)-tert.-butyl-benzoesäure und 15 Teile 4-tert.-Butylphenol werden analog den Angaben in Beispiel 21 umgesetzt. Ausbeute: 9 Teile 3-Pyrrolidino-7,5'(6')-di-tert.-butyl-fluoran der Formel

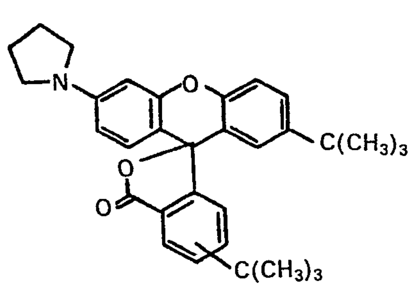

Schmp. 246–248 °C. Mit sauer reagierenden Substanzen erhält man eine Rotorangefärbung.

Beispiel 30

37 Teile 2-(2'-Hydroxy-4'-pyrrolidino-benzoyl)-4(5)-tert.-butyl-benzoesäure und 14,5 Teile β-Naphthol werden in 250 Teilen 50%iger Schwefelsäure 8 Stunden unter Rückfluss erhitzt. Die Aufarbeitung des Reaktionsgemisches erfolgt wie in Beispiel 21 beschrieben. Ausbeute: 6 Teile 3-Pyrrolidino- 7,8-benzo-5'(6')-tert.-butyl-fluoran der Formel

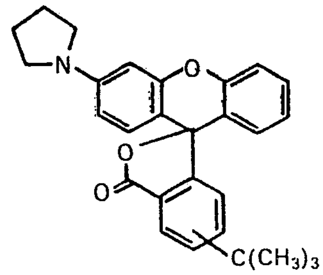

Beispiel 31

19 Teile 2-(2'-Hydroxy-4'-morpholinobenzoyl)-4(5)-tert.-butyl-benzoesäure und 7,2 Teile β-Naphthol werden analog den Angaben in Beispiel 21 umgesetzt. Ausbeute: 7 Teile 3-Morpholino-7,8-benzo-5'(6')-tert.-butyl-fluoran der Formel

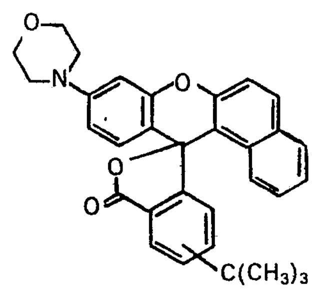

Schmp. 260–267 °C. Auf Elektronenakzeptorsubstanzen erhält man eine Rotfärbung.

Beispiele 32 bis 67

Analog den Angaben in Beispiel 21 werden Farbbildner der allgemeinen Formel Ib

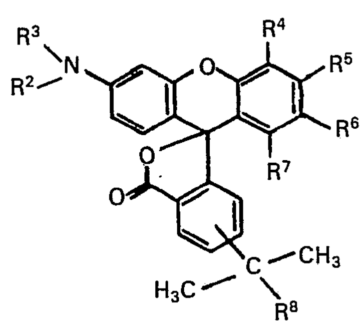

(Ib)

hergestellt. Die Bedeutung der Substituenten $R^2$ bis $R^8$ ist in der folgenden Tabelle angegeben.

Auf Elektronenakzeptorsubstanzen erhält man Färbungen mit den in der rechten Spalte angegebenen Farbtönen.

| Bsp. Nr. | $R^2$ | $R^3$ | $R^4$ | $R^5$ | $R^6$ | $R^7$ | $R^8$ | Farbton |
|---|---|---|---|---|---|---|---|---|
| 32 | $C_3H_7(n)$ | $C_3H_7(n)$ | H | H | $CH_3$ | H | $CH_3$ | rotorange |
| 33 | $C_4H_9(n)$ | $C_4H_9(n)$ | H | H | $CH_3$ | H | $CH_3$ | rotorange |
| 34 | | $-(CH_2)_5-$ | H | H | $CH_3$ | H | $CH_3$ | rotorange |
| 35 | $CH_3$ | $CH_3$ | $-CH{=}CH$ | $-CH{=}CH-$ | H | H | $CH_3$ | rot |
| 36 | $CH_3$ | $CH_3$ | H | $CH_3$ | Cl | $CH_3$ | $CH_3$ | rot |
| 37 | | $-(CH_2)_4-$ | H | $C_2H_5$ | H | $C_2H_5$ | $CH_3$ | rotorange |
| 38 | $CH_3$ | $CH_3$ | H | H | $CH_3$ | H | $CH_3$ | rotorange |
| 39 | $CH_3$ | $CH_3$ | H | H | $CH_3$ | H | $CH_3$ | rotorange |
| 40 | $-(CH_2)_2$ | $O(CH_2)_2-$ | H | H | $C(CH_3)_3$ | H | $CH_3$ | rotorange |
| 41 | $CH_3$ | $CH_3$ | H | $C(CH_3)_3$ | $CH_3$ | $C(CH_3)_3$ | $CH_3$ | rotorange |
| 42 | $C_2H_4OCH_3$ | $C_2H_4OCH_3$ | H | H | $CH_3$ | H | $CH_3$ | rotorange |
| 43 | $CH_3$ | $C_6H_5$ | H | H | $CH_3$ | H | $CH_3$ | rotorange |
| 44 | $CH_3$ | p-$C_6H_4-CH_3$ | H | H | $CH_3$ | H | $CH_3$ | rotorange |
| 45 | $CH_3$ | c–$C_6H_{11}$* | H | H | $CH_3$ | H | $CH_3$ | rotorange |
| 46 | $C_2H_5$ | c–$C_6H_{11}$ | H | H | $CH_3$ | H | $CH_3$ | rotorange |
| 47 | $C_2H_5$ | $C_2H_5$ | H | H | $C_6H_5$ | H | $CH_3$ | rotorange |
| 48 | $C_2H_5$ | $C_2H_5$ | H | H | 7,8–C H=CH–CH=CH–<br>\|<br>$NH_2$ | | $CH_3$ | blau |
| 49 | $C_2H_5$ | $C_2H_5$ | H | H | 7,8–C =CH–CH=CH–<br>\|<br>N–$CH_2C_6H_5$<br>H | | $CH_3$ | blau |

*c = cyclo

| Bsp. Nr. | $R^2$ | $R^3$ | $R^4$ | $R^5$ | $R^6$ | $R^7$ | $R^8$ | Farbton |
|---|---|---|---|---|---|---|---|---|
| 50 | $C_2H_5$ | $C_2H_5$ | H | H | 7,8–C =CH–CH=CH–<br>\|<br>$N(CH_2C_6H_5)_2$ | | $CH_3$ | blau |
| 51 | $C_2H_5$ | $C_2H_5$ | H | H | 7,8–C =CH–CH=CH–<br>\|<br>$N(CH_3)_2$ | | $CH_3$ | blau |
| 52 | $C_2H_5$ | $C_2H_5$ | H | H | $C_8H_{17}(n)$ | H | $CH_3$ | rotorange |
| 53 | $C_2H_5$ | $C_2H_5$ | H | H | $C_9H_{19}(n)$ | H | $CH_3$ | rotorange |
| 54 | $CH_3$ | p–$C_6H_4$–Cl | H | H | $CH_3$ | H | $CH_3$ | rotorange |
| 55 | $CH_2C_6H_5$ | $CH_2C_6H_5$ | H | H | $CH_3$ | H | $CH_3$ | rotorange |
| 56 | $C_2H_5$ | $C_2H_5$ | i–$C_3H_7$ | H | H | $CH_3$ | $CH_3$ | rotorange |
| 57 | 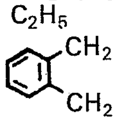 | | H | H | $CH_3$ | H | $CH_3$ | rotorange |
| 58 | $C_2H_5$ | $C_2H_5$ | H | $C(CH_3)_3$ | $CH_3$ | $C(CH_3)_3$ | $CH_3$ | rotorange |
| 59 | $C_2H_5$ | $C_2H_5$ | H | H | $CH_3$<br>\|<br>$CHC_2H_5$ | H | $CH_3$ | rotorange |
| 60 | $C_2H_5$ | $C_2H_5$ | Cl | H | Cl | H | $CH_3$ | rotorange |
| 61 | $C_2H_5$ | $C_2H_5$ | H | H | $CH_3$ | H | $C_2H_5$ | rotorange |
| 62 | $C_2H_5$ | $C_2H_5$ | H | H | $CH_3$ | H | $CH_2C(CH_3)_3$ | rotorange |
| 63 | $C_2H_5$ | $C_2H_5$ | H | H | $C_2H_4C_6H_5$ | H | $CH_3$ | rotorange |
| 64 | $C_2H_5$ | $C_2H_5$ | H | H | Br | H | $CH_3$ | rot |
| 65 | $C_2H_5$ | $C_2H_5$ | Cl | H | Cl | H | $CH_3$ | rot |
| 66 | $C_2H_5$ | $C_2H_5$ | H | H | $OCH_3$ | H | $CH_3$ | rot |
| 67 | | $-(CH_2)_5-$ | H | Cl | H | H | $CH_3$ | rot |

Beispiele 68 bis 75

Analog den Angaben in Beispiel 21 werden Farbbilder der allgemeinen Formel XI

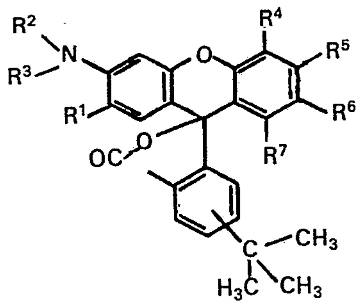

(XI)

hergestellt. Die Bedeutung der Substituenten $R^2$ bis $R^7$ ist in der folgenden Tabelle angegeben.

Auf Elektronenakzeptorsubstanzen erhält man Färbungen mit den in der rechten Spalte angegebenen Farbtönen.

| Bsp. Nr. | $R^2$ | $R^3$ | $R^4$ | $R^5$ | $R^6$ | $R^7$ | Farbton | Schmp. [°C] |
|---|---|---|---|---|---|---|---|---|
| 68 | $-(CH_2)_2$ | $-O-(CH_2)_2$ | H | $CH_3$ | $CH_3$ | H | rot | 90– 92 |
| 69 | $CH_3$ | $CH_3$ | H | $CH_3$ | Cl | H | rot | 188–190 |
| 70 | $-(CH_2)_2$ | $-O-(CH_2)_2$ | H | $CH_3$ | Cl | H | rot | 198–200 |
| 71 | $-(CH_2)_5$ | | $CH_3$ | H | $CH_3$ | H | rot | 214–216 |
| 72 | $-(CH_2)_5$ | | H | $CH_3$ | Cl | H | rot | 232–234 |
| 73 | $-(CH_2)_5-$ | | H | $CH_3$ | H | $CH_3$ | rot | 199–201 |
| 74 | $-(CH_2)_5$ | | H | H | Cl | H | rot | 204–206 |
| 75 | $-(CH_2)_5$ | | H | Cl | H | H | rot | 206–208 |

Beispiel 76

16 Teile 2-(2'-Hydroxy-3', 5'-dimethyl-benzoyl)-4(5)-tert.-butylbenzoesäure und 8 Teile 3-Ethylamino-4-methyl-phenol werden analog den Angaben in Beispiel 1 zum Farbbildner der Formel

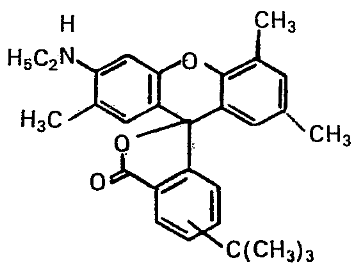

umgesetzt. Ausbeute: 9 Teile 2,5,7-Trimethyl-3-ethylamino-5'(6')-tert.-butylfluoran, Schmelzpunkt 258–260 °C. Die Verbindung entwickelt mit Elektronenakzeptorsubstanzen eine Orangefärbung.

Beispiel 77

18 Teile 2-(2'-Hydroxy-4'-methyl-5'-chlorbenzoyl)-4(5)-tert.-butylbenzoesäure und 8 Teile 3-Ethylamino-4-methyl-phenol werden analog den Angaben in Beispiel 1 zum Farbbildner der Formel

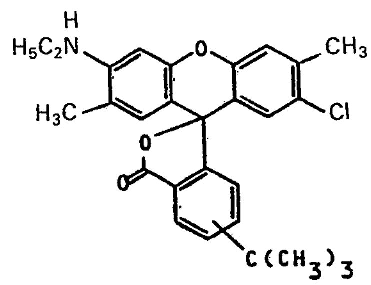

umgesetzt. Ausbeute: 7 Teile 7-Chlor-2,6-dimethyl-3-ethylamino-5'(6')-tert.-butylfluoran, Schmelzpunkt 186–188 °C. Die Substanz entwickelt mit Elektronenakzeptorsubstanzen eine Orangefärbung.

Beispiel 78

24 Teile 2-(2'-Hydroxy-4'-diethylaminobenzoyl)-4(5)-tert.-butylbenzoesäure und 10,15 Teile 2-Methyl-4-methoxy-diphenylamin werden 36 Stunden lang bei Raumtemperatur in 74 Teilen 96%iger Schwefelsäure gerührt. Nach Eintragen der Reaktionslösung in 750 Teile Eiswasser wird der entstandene Niederschlag abgesaugt und mit Wasser neutral gewaschen. Das Produkt wird in einem Gemisch aus 250 Teilen Toluol und 250 Teilen 10%iger Natronlauge bei 90 °C gelöst. Nach Abtrennen der wässrigen Phase wird die Toluollösung zweimal mit 150 Teilen 10%iger Natronlauge und einmal mit 5%iger Kochsalzlösung ausgeschüttelt. Die organische Phase wird auf die Hälfte ihres Volumens eingeengt, wobei 18 Teile 3-Diethylamino-6-methyl-7-phenylamino-5'(6')-tert.-butylfluoran der Formel

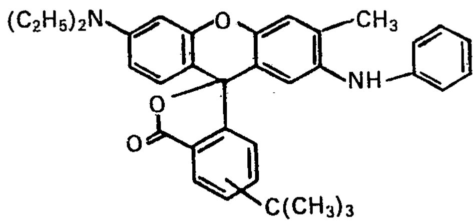

auskristallisieren. Die Verbindung schmilzt bei 247–250 °C und gibt in Kontakt mit Elektronenakzeptorsubstanzen eine intensive Schwarzfärbung.

Beispiele 79 bis 102

Unter Beibehaltung der Molverhältnisse wurden analog Beispiel 78 Farbbildner der allgemeinen Formel XII

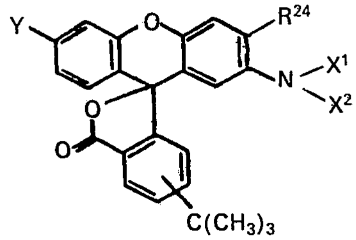

(XII)

erhalten. Die Bedeutung der Substituenten ist in der Tabelle angegeben:

| Bsp. Nr. | Y | $R^{24}$ | $X^2$ | $X^1$ | Schmp. °C | Farbton |
|---|---|---|---|---|---|---|
| 79 | $(CH_3)_2N$ | $CH_3$ | 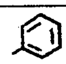 | H | 185–189 | schwarz |
| 80 | 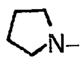 | $CH_3$ | | H | 245–248 | schwarz |
| 81 | 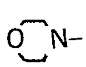 | $CH_3$ | 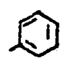 | H | 218–220 | grünschwarz |
| 82 | $(C_2H_5)_2N$ | H | 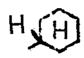 | H | 151–155 | oliv |
| 83 | $(C_2H_5)_2N$ | H | $CH{<}^{CH_3}_{CH_3}$ | H | 218–221 | oliv |
| 84 | $(C_2H_5)N$ | $CH_3$ | 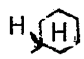 | H | 172–175 | oliv |
| 85 | 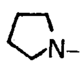 | $CH_3$ | 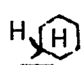 | H | 225–231 | oliv |
| 86 | 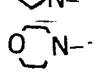 | $CH_3$ | 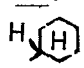 | H | 267–273 | oliv |
| 87 | 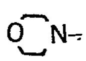 | $CH_3$ | $CH{<}^{CH_3}_{CH_3}$ | H | 162–165 | oliv |
| 88 | 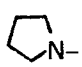 | $CH_3$ | H | H | | violett |
| 89 | 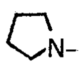 | H | $C_2H_4$– 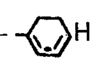 | H | | oliv |
| 90 | 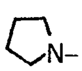 | H | $CH_2$– 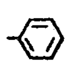 | H | | oliv |
| 91 | 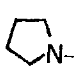 | H | $CH(CH_3)_2$ | H | | oliv |
| 92 | 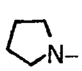 | $CH_3$ | $CH(CH_3)_2$ | H | | schwarz |
| 93 | 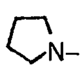 | H | $-(CH_2)_4-$ | | | grün |
| 94 | 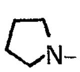 | H | $-CH_2)_5$ | | | violett |

| Bsp. Nr. | Y | $R^{24}$ | $X^2$ | $X^1$ | Schmp. °C | Farbton |
|---|---|---|---|---|---|---|
| 95 | $N(C_2H_5)_2$ | Cl | $CH(CH_3)_2$ | H | | schwarz |
| 96 | $N(C_2H_5)_2$ | Cl | 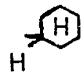 | H | | schwarz |
| 97 | $N(CH_3)_2$ | $CH_3$ | $CH(CH_3)_2$ | H | | schwarz |
| 98 | $N(CH_3)_2$ | $CH_3$ | 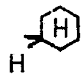 | H | | schwarz |
| 99 | 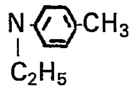 | H | 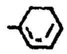 | $CH_3$ | | grün |
| 100 | 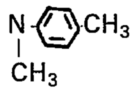 | $CH_3$ | 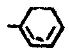 | H | | schwarz |
| 101 | 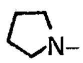 | H | 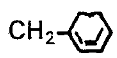 | 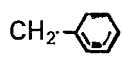 | | grün |
| 102 | $N(C_2H_5)_2$ | 5–Cl | 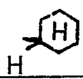 | H | | oliv |

Beispiel 103

23 Teile 2-(2'-Hydroxy-4'-pyrrolidino-benzoyl)-4(5)-tert.-butylbenzoesäure und 8,5 Teile 2-Methyl-4-methoxy-isopropylanilin werden in 74 Teilen 90%iger Schwefelsäure in 6 Stunden bei 70 °C umgesetzt. Der Ansatz wird wie in Beispiel 78 aufgearbeitet. Dabei werden 10 Teile Farbbildner der Formel

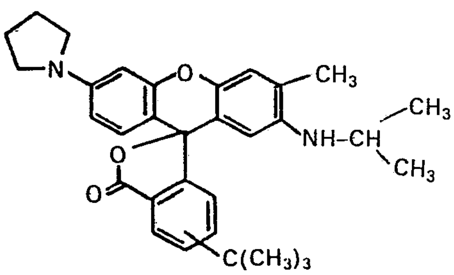

erhalten. Die Verbindung gibt in Kontakt mit Elektronenakzeptorsubstanzen eine olive Färbung.

Nach der gleichen Arbeitsweise werden Farbbildner der allgemeinen Formel XII mit den folgenden Resten gewonnen:

| Bsp. Nr. | Y | $R^{24}$ | $X^2$ | $X^1$ | Farbton |
|---|---|---|---|---|---|
| 104 | $(CH_3)N$ | H | $n–C_8H_{17}$ | H | oliv |
| 105 | $(CH_3)_2N$ | H | 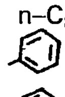 | H | oliv |
| 106 | $(CH_3)_2N$ | H | 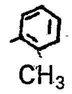 | H | oliv |
| 107 | $(C_2H_5)N$ | $CH_3$ | $CH_3$ | H | oliv |
| 108 | $(C_2H_5)N$ | $CH_3$ | $C_2H_5$ | H | oliv |
| 109 | $(C_2H_5)N$ | $CH_3$ | $n–C_4H_9$ | H | oliv |
| 110 | $CH_3$ 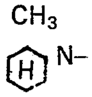 | H | $C_2H_5$ | H | oliv |

| Bsp. Nr. | Y | $R^{24}$ | $X^2$ | $X^1$ | Farbton |
|---|---|---|---|---|---|
| 111 | | $CH_3$ | $C_2H_5$ | H | oliv |
| 112 | $(CH_3)_2N$ | H | $CH_2C_6H_5$ | $CH_2C_6H_5$ | oliv |
| 113 | $(C_2H_5)_2N$ | $CH_3$ | n–$C_3H_7$ | $CH_3$ | oliv |
| 114 | $(C_2H_5)_2N$ | $CH_3$ | H | H | rotbraun |

**Patentansprüche**

1. Fluorane der allgemeinen Formel I

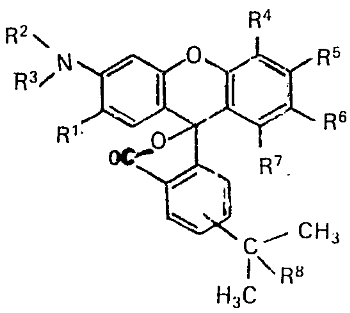

(I),

in der

$R^1$ für Wasserstoff oder Methyl,

$R^2$ für Wasserstoff, $C_1$- bis $C_4$-Alkyl, $C_7$- oder $C_8$-Phenalkyl, Cyanethyl, Chlorethyl oder Alkoxyalkyl mit insgesamt 3 bis 5 C-Atomen,

$R^3$ für Wasserstoff, $C_1$- bis $C_4$-Alkyl, $C_7$- bis $C_{10}$-Phenalkyl, $C_5$- oder $C_6$-Cycloalkyl, Cyanethyl, Chlorethyl, Alkoxyalkyl mit insgesamt 3 bis 5 C-Atomen oder für gegebenenfalls durch $C_1$- bis $C_4$-Alkyl, Methoxy, Chlor oder Brom substituiertes Phenyl, oder $-N\langle^{R^2}_{R^3}$ für N-Pyrrolidinyl, N-Piperidinyl, N-Morpholinyl oder N-Isoindolinyl,

$R^4$ und $R^5$ unabhängig voneinander für Wasserstoff, $C_1$- bis $C_4$-Alkyl, $C_1$- bis $C_4$-Alkoxy oder Halogen,

$R^6$ für Wasserstoff, Halogen, $C_1$- bis $C_{12}$-Alkyl, $C_1$- bis $C_4$-Alkoxy, $C_7$- bis $C_{10}$-Phenalkoxy, Phenoxy, Phenyl oder für eine Gruppe der Formel

$$-N\langle^{X^1}_{X^2},$$

worin $X^1$ Wasserstoff, $C_1$- bis $C_4$-Alkyl, Chlorethyl, Alkoxyalkyl mit insgesamt 3 bis 5 C-Atomen, $C_7$- oder $C_8$-Phenalkyl und $X^2$ Wasserstoff, $C_1$- bis $C_{12}$-Alkyl, Chlorethyl, $C_5$- oder $C_6$-Cycloalkyl, Alkoxyalkyl mit insgesamt 3 bis 5 C-Atomen, $C_7$- bis $C_{10}$-Phenalkyl oder gegebenenfalls durch Halogen, $C_1$- bis $C_4$-Alkyl, $C_1$- bis $C_4$-Alkoxy, $C_1$- bis $C_4$-Alkylcarbonyl, Benzoyl oder Phenoxy substituiertes Phenyl, $C_1$- bis $C_4$-Alkoxycarbonyl-$C_1$- bis $C_3$-alkyl oder Carbamoyl-$C_1$- bis $C_3$-alkyl bedeuten, oder $N\langle^{X^1}_{X^2}$ N-Pyrrolidinyl, N-Piperidinyl oder N-Morpholinyl bedeuten,

$R^7$ für Wasserstoff, $C_1$- bis $C_4$-Alkyl oder Halogen und

$R^8$ für $C_1$- bis $C_5$-Alkyl stehen und wobei die Reste $R^4$ und $R^5$ und/oder $R^6$ und $R^7$ gemeinsam je eine –CH=CH–CH=CH-Brücke sein können und der ankondensierte Ring gegebenenfalls ein Halogen oder eine gegebenenfalls durch $C_1$- bis $C_4$-Alkyl, $C_7$- oder $C_8$-Phenalkyl mono- oder disubstituierte Aminogruppe tragen kann.

2. Fluorane gemäss Anspruch 1, dadurch gekennzeichnet, dass $R^8$ für Methyl, Ethyl oder $-CH_2-C(CH_3)_3$ steht.

3. Fluorane gemäss Anspruch 1, gekennzeichnet durch die allgemeine Formel II

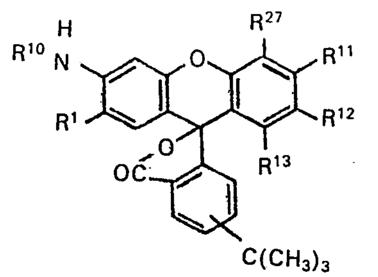

(II)

in der

$R^1$ für Wasserstoff oder Methyl,

$R^{10}$ für Wasserstoff, $C_1$- bis $C_4$-Alkyl, Cyclohexyl, Benzyl oder für gegebenenfalls durch ein oder zwei Methyl und/oder Ethyl und/oder ein Chlor, Brom oder Methoxy substituiertes Phenyl,

$R^{11}$ für Wasserstoff, Chlor oder $C_1$- bis $C_4$-Alkyl,

$R^{12}$ für $C_1$- bis $C_{12}$-Alkyl oder Halogen,

$R^{13}$ für Wasserstoff oder Methyl und

$R^{27}$ für Wasserstoff oder Methyl stehen.

4. Fluorane gemäss Anspruch 3, dadurch gekennzeichnet, dass $R^1$ für Wasserstoff und $R^{10}$ für ein durch ein oder zwei Methyl und/oder Ethyl oder für ein durch ein Chlor, Brom oder Methoxy substituiertes Phenyl stehen.

5. Fluorane gemäss Anspruch 1, gekennzeichnet durch die allgemeine Formel III

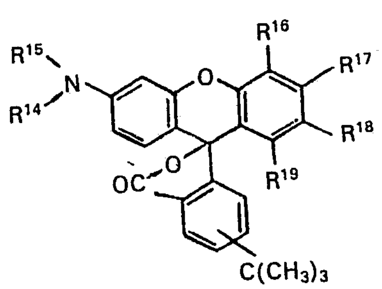

(III)

in der

$R^{14}$ für $C_1$- bis $C_4$-Alkyl, $C_7$- oder $C_8$-Phenalkyl oder Alkoxyalkyl mit insgesamt 3 bis 5 C-Atomen,

$R^{15}$ für $C_1$- bis $C_4$-Alkyl, $C_7$- bis $C_{10}$-Phenalkyl, $C_5$- oder $C_6$-Cycloalkyl, Alkoxyalkyl mit insgesamt 3 bis 5 C-Atomen oder für gegebenenfalls durch $C_1$- bis $C_4$-Alkyl, Chlor oder Brom einfach substituiertes Phenyl oder – 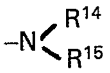 für N-Pyrrolidinyl, N-Piperidinyl oder N-Morpholinyl,

$R^{16}$ und $R^{17}$ unabhängig voneinander für Wasserstoff, Chlor oder $C_1$- bis $C_4$-Alkyl,

$R^{18}$ für Wasserstoff, $C_1$- bis $C_{12}$-Alkyl, $C_1$- bis $C_4$-Alkoxy, $C_7$- bis $C_{10}$-Phenalkoxy, Phenoxy oder Halogen und

$R^{19}$ für Wasserstoff oder $C_1$- bis $C_4$-Alkyl stehen.

6. Fluorane gemäss Anspruch 1, gekennzeichnet durch die allgemeine Formel IV

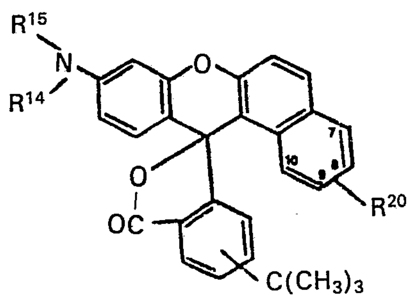

(IV)

in der

$R^{14}$ für $C_1$- bis $C_4$-Alkyl, $C_7$- oder $C_8$-Phenalkyl oder Alkoxyalkyl mit insgesamt 3 bis 5 C-Atomen,

$R^{15}$ für $C_1$- bis $C_4$-Alkyl, $C_7$- bis $C_{10}$-Phenalkyl, $C_5$- oder $C_6$-Cycloalkyl, Alkoxyalkyl mit insgesamt 3 bis 5 C-Atomen oder für gegebenenfalls durch $C_1$- bis $C_4$-Alkyl, Chlor oder Brom einfach substituiertes Phenyl oder – 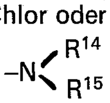 für N-Pyrrolidinyl, N-Piperidinyl oder N-Morpholinyl,

$R^{20}$ für Wasserstoff oder für eine gegebenenfalls durch Methyl, Ethyl oder Benzyl mono- oder disubstituierte Aminogruppe in der 7-, 9- oder 10-Stellung stehen.

7. Fluorane gemäss Anspruch 1, gekennzeichnet durch die allgemeine Formel V

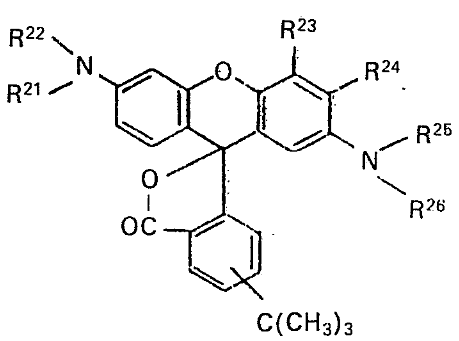

(V)

in der

$R^{21}$ für $C_1$- bis $C_4$-Alkyl, $C_7$- oder $C_8$-Phenalkyl,

$R^{22}$ für $C_1$- bis $C_4$-Alkyl, $C_7$- bis $C_{10}$-Phenalkyl, $C_5$- oder $C_6$-Cycloalkyl oder durch $C_1$- bis $C_4$-Alyl oder Chlor substituiertes Phenyl oder – 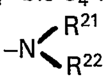 für N-Pyrrolidinyl, N-Piperidinyl oder N-Morpholinyl,

$R^{23}$ für Wasserstoff oder Chlor,

$R^{24}$ für Wasserstoff oder Methyl, wobei wenn $R^{23}$ Wasserstoff ist, $R^{24}$ auch Chlor sein kann,

$R^{25}$ für Wasserstoff, $C_1$- bis $C_4$-Alkyl, $C_7$- oder $C_8$-Phenalkyl, und

$R^{26}$ für Wasserstoff, $C_1$- bis $C_{12}$-Alkyl, $C_7$- bis $C_{10}$-Phenalkyl, $C_5$- oder $C_6$-Cycloalkyl oder für eine gegebenenfalls durch Chlor, $C_1$- bis $C_4$-Alkyl, Methoxy, Ethoxy, $C_1$- bis $C_2$-Alkylcarbonyl, Benzoyl oder Phenoxy substituiertes Phenyl oder – 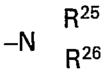 für N-Pyrrolidinyl, N-Piperidinyl oder N-Morpholinyl stehen.

8. Verwendung der Fluorane gemäss den Ansprüchen 1 bis 7 als Farbbildner in druck- oder wärmeempfindlichen Aufzeichnungssystemen.

## Revendications

1. Fluorannes de formule générale I

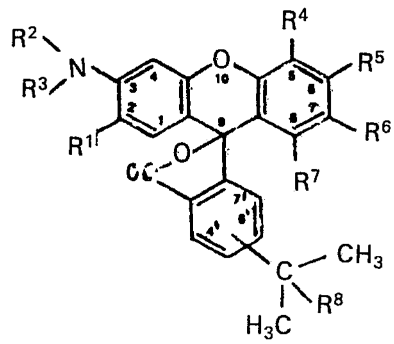

(I),

dans laquelle

$R^1$ est mis pour un atome d'hydrogène ou un radical méthyle,

$R^2$ est mis pour un atome d'hydrogène, un radical alkyle en $C_1$ à $C_4$, phénalkyle en $C_6$ ou $C_7$, cyanéthyle, chloréthyle ou alcoxyalkyle contenant au total 3 à 5 atomes de C,

$R^3$ est mis pour un atome d'hydrogène, un radical alkyle en $C_1$ à $C_4$, phénalkyle en $C_7$ à $C_{10}$, cycloalkyle en $C_5$ ou $C_6$, cyanéthyle, chloréthyle, alcoxyalkyle contenant au total 3 à 5 atomes de C ou pour un radical phényle éventuellement substitué par un radical alkyle en $C_1$ à $C_4$, méthoxy, un atome de chlore ou de brome, ou – 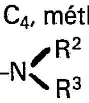 est mis pour un groupement N-pyrrolidinyle, N-pipéridinyle, N-morpholinyle ou N-isoindolinyle,

$R^4$ et $R^5$, indépendamment l'un de l'autre, sont mis chacun pour un atome d'hydrogène, un radical alkyle en $C_1$ à $C_4$, alcoxy en $C_1$ à $C_4$ ou un atome d'halogène,

$R^6$ est mis pour un atome d'hydrogène, d'halogène, un radical alkyle en $C_1$ à $C_{12}$, alcoxy en $C_1$ à $C_4$, phénalcoxy en $C_7$ à $C_{10}$, phénoxy, phényle ou pour un groupe de formule

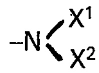

dans laquelle $X^1$ représente un atome d'hydrogène, un radical alkyle en $C_1$ à $C_4$, chloréthyle, alcoxyalkyle contenant au total 3 à 5 atomes de C, phénalkyle en $C_7$ ou $C_8$ et $X^2$ représente un atome d'hydrogène, un radical alkyle en $C_1$ à $C_{12}$, chloréthyle, cycloalkyle en $C_5$ ou $C_6$, alcoxyalkyle contenant au total 3 à 5 atomes de C, phénalkyle en $C_7$ à $C_{10}$ ou phényle éventuellement substitué par un atome d'halogène, un radical alkyle en $C_1$ à $C_4$, alcoxy en $C_1$ à $C_4$, alkylcarbonyle en $C_1$ à $C_4$, benzoyle ou phénoxy, un radical $C_1$–$C_4$-alcoxycarbonyl-$C_1$–$C_3$-alkyle ou carbamoyl-$C_1$–$C_3$-alkyle, ou 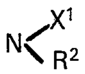 représente un radical N-pyrrolidinyle, N-pipéridinyle ou N-morpholinyle,

$R^7$ est mis pour un atome d'hydrogène, un radical alkyle en $C_1$ à $C_4$ ou un atome d'halogène et

$R^8$ est mis pour un radical alkyle en $C_1$ à $C_5$, les radicaux $R^4$ et $R^5$ et/ou $R^6$ et $R^7$ pouvant former en commun un pont $-CH=CH-CH=CH-$ et le noyau condensé pouvant éventuellement porter un atome d'halogène ou un groupe amino éventuellement mono- ou bisubstitué par un radical alkyle en $C_1$ à $C_4$ ou phénalkyle en $C_7$ ou $C_8$.

2. Fluorannes selon la revendication 1, caractérisés en ce que $R^8$ est mis pour un radical méthyle, éthyle ou $-CH_2-C(CH_3)_3$.

3. Fluorannes selon la revendication 1, caractérisés par la formule générale II

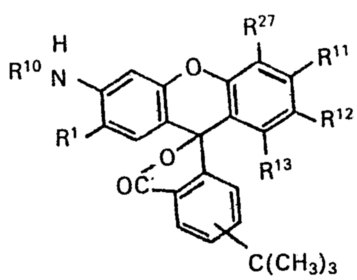

(II),

dans laquelle

$R^1$ est mis pour un atome d'hydrogène ou un radical méthyle,

$R^{10}$ est mis pour un atome d'hydrogène, un radical alkyle en $C_1$ à $C_4$, cyclohexyle, benzyle ou pour un radical phényle éventuellement substitué par un ou deux radicaux méthyle et/ou un atome de chlore, de brome ou un radical méthoxy,

$R^{11}$ est mis pour un atome d'hydrogène, de chlore ou un radical alkyle en $C_1$ à $C_4$,

$R^{12}$ est mis pour un radical alkyle en $C_1$ à $C_{12}$ ou un atome d'halogène,

$R^{13}$ est un atome d'hydrogène ou un radical méthyle et

$R^{27}$ est mis pour un atome d'hydrogène ou un radical méthyle.

4. Fluorannes selon la revendication 3, caractérisés en ce que $R^1$ est mis pour un atome d'hydrogène et $R^{10}$ pour un radical phényle substitué par un ou deux radicaux méthyle et/ou éthyle ou substitué par un atome de chlore, de brome ou un radical méthoxy.

5. Fluorannes selon la revendication 1, caractérisés par la formule générale III

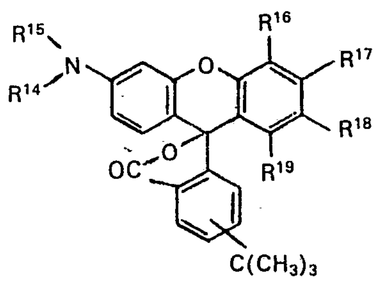

(III),

dans laquelle

$R^{14}$ est mis pour un radical alkyle en $C_1$ à $C_4$, phénalkyle en $C_7$ ou $C_8$ ou alcoxyalkyle contenant 3 à 5 atomes de C au total,

$R^{15}$ est mis pour un radical alkyle en $C_1$ à $C_4$, phénalkyle en $C_7$ à $C_{10}$, cycloalkyle en $C_5$ ou $C_6$, alcoxyalkyle contenant 3 à 5 atomes de C au total ou pour un radical phényle éventuellement substitué une fois par un radical alkyle en $C_1$ à $C_4$, un atome de chlore ou de brome, ou 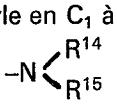 est mis pour un radical N-pyrrolidinyle, N-pipéridinyle ou N-morpholinyle,

$R^{16}$ et $R^{17}$, indépendamment l'un de l'autre, sont mis chacun pour un atome d'hydrogène, de chlore ou un radical alkyle en $C_1$ à $C_4$,

$R^{18}$ est mis pour un atome d'hydrogène, un radical alkyle en $C_1$ à $C_{12}$, alcoxy en $C_1$ à $C_4$, phénalcoxy en $C_7$ à $C_{10}$, phénoxy ou un atome d'halogène, et

$R^{19}$ est mis pour un atome d'hydrogène ou un radical alkyle en $C_1$ à $C_4$.

6. Fluorannes selon la revendication 1, caractérisés par la formule générale IV

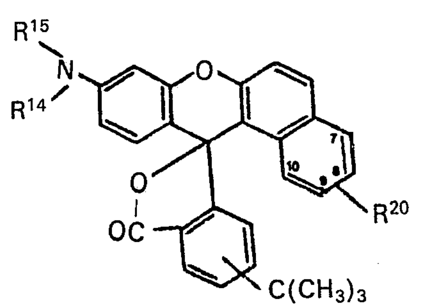

(IV),

dans laquelle

$R^{14}$ est mis pour un radical alkyle en $C_1$ à $C_4$, phénalkyle en $C_7$ ou $C_8$ ou alcoxyalkyle contenant 3 à 5 atomes de C au total,

$R^{15}$ est mis pour un radical alkyle en $C_1$ à $C_4$, phénalkyle en $C_7$ à $C_{10}$, cycloalkyle en $C_5$ ou $C_6$, alcoxyalkyle contenant 3 à 5 atomes de C au total ou pour un radical phényle éventuellement substitué une fois par un radical alkyle en $C_1$ à $C_4$, un atome de chlore ou de brome, ou $-N\left\langle\begin{matrix}R^{14}\\R^{15}\end{matrix}\right.$ est mis pour un radical N-pyrrolidinyle, N-pipéridinyle ou N-morpholinyle,

$R^{20}$ est mis pour un atome d'hydrogène ou, dans la position 7, 9 ou 10, pour un groupe amino éventuellement mono- ou bisubstitué par des radicaux méthyle, éthyle ou benzyle.

7. Fluorannes selon la revendication 1, caractérisés par la formule générale V

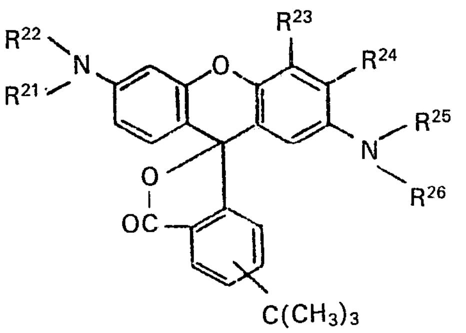

(V),

dans laquelle

$R^{21}$ est mis pour un radical alkyle en $C_1$ à $C_4$, phénalkyle en $C_7$ ou $C_8$,

$R^{22}$ est mis pour un radical alkyle en $C_1$ à $C_4$, phénalkyle en $C_7$ à $C_{10}$, cycloalkyle en $C_5$ ou $C_6$ ou phényle substitué par un radical alkyle en $C_1$ à $C_4$ ou un atome de chlore, ou $-N\left\langle\begin{matrix}R^{21}\\R^{22}\end{matrix}\right.$ est mis pour un radical N-pyrrolidinyle, N-pipéridinyle ou N-morpholinyle,

$R^{23}$ est mis pour un atome d'hydrogène ou de chlore,

$R^{24}$ est mis pour un atome d'hydrogène ou un radical méthyle, $R^{24}$ pouvant être aussi un atome de chlore quand $R^{23}$ est un atome d'hydrogène,

$R^{25}$ est mis pour un atome d'hydrogène, un radical alkyle en $C_1$ à $C_4$ ou phénalkyle en $C_7$ ou $C_8$, et

$R^{26}$ est mis pour un atome d'hydrogène, un radical alkyle en $C_1$ à $C_{12}$, phénalkyle en $C_7$ à $C_{10}$, cycloalkyle en $C_5$ ou $C_6$ ou pour un radical phényle éventuellement substitué par un atome de chlore, un radical alkyle en $C_1$ à $C_4$, méthoxy, éthoxy, $C_1$-$C_2$-alkylcarbonyle, benzoyle ou phénoxy, ou $-N\left\langle\begin{matrix}R^{25}\\R^{26}\end{matrix}\right.$ est mis pour un radical N-pyrrolidinyle, N-pipéridinyle ou N-morpholinyle.

8. Utilisation des fluorannes selon l'une quelconque des revendications 1 à 7 comme chromogènes dans les systèmes d'enregistrement sensibles à la pression ou à la chaleur.

## Claims

1. A fluoran of the general formula I

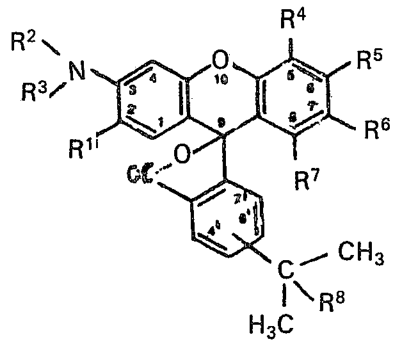

(I),

where

$R^1$ is hydrogen or methyl,

$R^2$ is hydrogen, $C_1$-$C_4$-alkyl, $C_7$- or $C_8$-phenylalkyl, cyanoethyl, chloroethyl or alkoxyalkyl having a total of 3 to 5 carbon atoms,

$R^3$ is hydrogen, $C_1$-$C_4$-alkyl, $C_7$-$C_{10}$-phenylalkyl, $C_5$- or $C_6$-cycloalkyl, cyanoethyl, chloroethyl, alkoxyalkyl having a total of 3 to 5 carbon atoms, or phenyl which is unsubstituted or substituted by $C_1$-$C_4$-alkyl, methoxy, chlorine or bromine, or $-N\left\langle\begin{matrix}R^{2}\\R^{3}\end{matrix}\right.$ is N-pyrrolidinyl, N-piperidinyl, N-morpholinyl or N-isoindolinyl,

$R^4$ and $R^5$ independently of one another are hydrogen, $C_1$-$C_4$-alkyl, $C_1$-$C_4$-alkoxy or halogen,

$R^6$ is hydrogen, halogen, $C_1$-$C_{12}$-alkyl, $C_1$-$C_4$-alkoxy, $C_7$-$C_{10}$-phenylalkoxy, phenoxy, phenyl, or a group of the formula

$$-N\left\langle\begin{matrix}X^{1}\\X^{2}\end{matrix}\right. ,$$

where $X^1$ is hydrogen, $C_1$-$C_4$-alkyl, chloroethyl, alkoxyalkyl having a total of 3 to 5 carbon atoms, $C_7$- or $C_8$-phenylalkyl, and $X^2$ is hydrogen, $C_1$-$C_{12}$-alkyl, chloroethyl, $C_5$- or $C_6$-cycloalkyl, alkoxyalkyl having a total of 3 to 5 carbon atoms, $C_7$-$C_{10}$-phenylalkyl, or phenyl which is unsubstituted or substituted by halogen, $C_1$-$C_4$-alkyl, $C_1$-$C_4$-alkoxy, $C_1$-$C_4$-alkylcarbonyl, benzoyl or phenoxy, or is $C_1$-$C_4$-alkoxycarbonyl-$C_1$-$C_3$-alkyl, or carbamyl-$C_1$-$C_3$-alkyl, or $-N\left\langle\begin{matrix}X^{1}\\X^{2}\end{matrix}\right.$ is N-pyrrolidinyl, N-piperidinyl or N-morpholinyl,

$R^7$ is hydrogen, $C_1$–$C_4$-alkyl or halogen, and

$R^8$ is $C_1$–$C_5$-alkyl, and the radicals $R^4$ and $R^5$ together and/or $R^6$ and $R^7$ together can each be a $-CH=CH-CH=CH-$ bridge, and the fused-on ring can be unsubstituted or substituted by halogen or by amino which is unsubstituted or monosubstituted or disubstituted by $C_1$–$C_4$-alkyl or $C_7$- or $C_8$-phenylalkyl.

2. A fluoran as claimed in claim 1, wherein $R^8$ is methyl, ethyl or $-CH_2-C(CH_3)_3$.

3. A fluoran as claimed in claim 1, of the general formula II

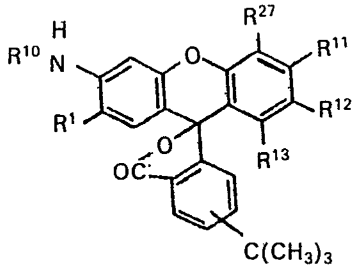

(II),

where

$R^1$ is hydrogen or methyl,

$R^{10}$ is hydrogen, $C_1$–$C_4$-alkyl, cyclohexyl or benzyl, or is phenyl which is unsubstituted or substituted by one or two methyl and/or ethyl groups and/or by one chlorine or bromine atom or one methoxy group.

$R^{11}$ is hydrogen, chlorine, or $C_1$–$C_4$-alkyl,

$R^{12}$ is $C_1$–$C_{12}$-alkyl or halogen, and

$R^{13}$ and $R^{27}$ are each hydrogen or methyl.

4. A fluoran as claimed in claim 3, wherein $R^1$ is hydrogen and $R^{10}$ is phenyl which is substituted by one or two methyl and/or ethyl groups or by one chlorine or bromine atom or one methoxy group.

5. A fluoran as claimed in claim 1, of the general formula III

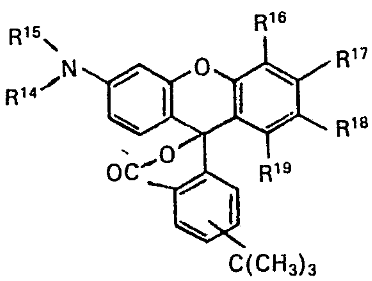

(III),

where

$R^{14}$ is $C_1$–$C_4$-alkyl, $C_7$- or $C_8$-phenylalkyl or alkoxyalkyl having a total of 3 to 5 carbon atoms,

$R^{15}$ is $C_1$–$C_4$-alkyl, $C_7$–$C_{10}$-phenylalkyl, $C_5$- or $C_6$-cycloalkyl or alkoxyalkyl having a total of 3 to 5 carbon atoms, or is phenyl which is unsubstituted or monosubstituted by $C_1$–$C_4$-alkyl, chlorine or bromine, or 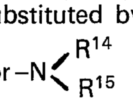 is N-pyrrolidinyl, N-piperidinyl or N-morpholinyl,

$R^{16}$ and $R^{17}$ independently of one another are hydrogen, chlorine or $C_1$–$C_4$-alkyl,

$R^{18}$ is hydrogen, $C_1$–$C_{12}$-alkyl, $C_1$–$C_4$-alkoxy, $C_7$–$C_{10}$-phenylalkoxy, phenoxy or halogen, and

$R^{19}$ is hydrogen or $C_1$–$C_4$-alkyl.

6. A fluoran as claimed in claim 1, of the general formula IV

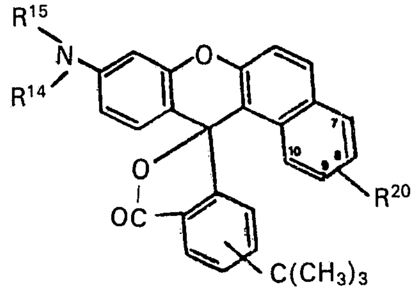

(IV),

where

$R^{14}$ is $C_1$–$C_4$-alkyl, $C_7$- or $C_8$-phenylalkyl or alkoxyalkyl having a total of 3 to 5 carbon atoms,

$R^{15}$ is $C_1$–$C_4$-alkyl, $C_7$–$C_{10}$-phenylalkyl, $C_5$- or $C_6$-cycloalkyl or alkoxyalkyl having a total of 3 to 5 carbon atoms, or is phenyl which is unsubstituted or monosubstituted by $C_1$–$C_4$-alkyl, chlorine or bromine, or 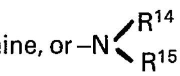 is N-pyrrolidinyl, N-piperidinyl or N-morpholinyl, and

$R^{20}$ is hydrogen, or amino which is unsubstituted or monosubstituted or disubstituted by methyl, ethyl or benzyl and is in the 7-, 9- or 10-position.

7. A fluoran as claimed in claim 1, of the general formula V

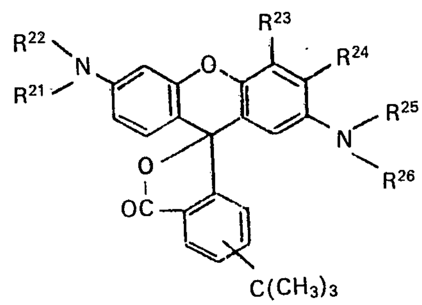

(V),

where

$R^{21}$ is $C_1$–$C_4$-alkyl or $C_7$- or $C_8$-phenylalkyl,

$R^{22}$ is $C_1$–$C_4$-alkyl, $C_7$–$C_{10}$-phenylalkyl or $C_5$- or $C_6$-cycloalkyl, or is phenyl which is substituted by $C_1$- $C_4$-alkyl or chlorine, or 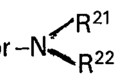 is N-pyrrolidinyl, N-piperidinyl or N-morpholinyl,

$R^{23}$ is hydrogen or chlorine,

$R^{24}$ is hydrogen or methyl, and, when $R^{23}$ is hydrogen, $R^{24}$ may furthermore be chlorine,

$R^{25}$ is hydrogen, $C_1$–$C_4$-alkyl or $C_7$- or $C_8$-phenylalkyl, and

$R^{26}$ is hydrogen, $C_1$–$C_{12}$-alkyl, $C_7$–$C_{10}$-phenylalkyl or $C_5$- or $C_6$-cycloalkyl, or is phenyl which is unsubstituted or substituted by chlorine, $C_1$–$C_4$-alkyl, methoxy, ethoxy, $C_1$- or $C_2$-alkylcarbonyl, benzoyl or phenoxy, or 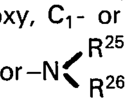 is N-pyrrolidinyl, N-piperidinyl or N-morpholinyl.

8. The use of a fluoran as claimed in claims 1 to 7 as colour former in pressure-sensitive and heat-sensitive recording systems.